# EUROPEAN PATENT SPECIFICATION

(11) **EP 3 551 260 B1**
(45) Date of publication and mention of the grant of the patent: **14.07.2021**
(21) Application number: 17878112.6
(22) Date of filing: 08.12.2017
(51) Int. Cl.: A61M 11/00, A61M 11/02, A61M 15/00, A61M 16/00

(54) **INHALER**
INHALATOR
INHALATEUR

(30) Priority: 09.12.2016 US 201662432237 P
(43) Date of publication of application: 16.10.2019
(73) Proprietor: MicroDose Therapeutx, Inc., Ewing, NJ 08628 (US)
(72) Inventor: KEIP, Jeffrey, D., Columbus, OH 43201 (US); MORRISON, Mark, Steven, Basking Ridge, NJ 07920 (US); WEITZEL, Douglas, E., Hamilton, NJ 08619 (US)
(74) Representative: Elkington and Fife LLP
(86) International application number: PCT/US2017/065289
(87) International publication number: WO 2018/107018

(56) References cited:
- EP-A1- 2 521 584
- EP-B1- 1 083 952
- EP-B1- 2 432 536
- WO-A1-2010/067240
- WO-A1-2016/033421
- US-A- 6 026 809
- US-A1- 2007 240 712
- US-A1- 2014 261 414
- US-A1- 2014 261 414
- US-A1- 2016 001 021
- US-A1- 2016 144 141
- US-A1- 2016 144 141
- US-B2- 8 567 394

## Description

### BACKGROUND

Metered dose inhalers (MDIs), dry powder inhalers, jet nebulizers, and ultrasonic nebulizers are medication delivery systems for treating respiratory diseases, such as asthma and chronic obstructive pulmonary disease (COPD). MDIs may utilize a "press and breath" medication delivery mechanism, whereby a user depresses a canister to release an aerosolized dose of medication before inhaling the dose from a mouthpiece on the device. To ensure a full dose of medication is received, MDIs may require the user to time the release of medication from the inhaler with the timing of his or her inhalation. Thus, MDIs may be susceptible to misoperation if the user is unable to properly and consistently time their inhalation when depressing the medication canister.

Other types of inhalers, such as dry powder inhalers, may be breath-actuated and may rely on a user's ability to generate a sufficient air flow within the device to activate the drug delivery mechanism and enable the user to receive the medication. If the user's inhalation is too weak, the device may not be able to aerosolize a full dose of medication. Thus, the ability of a breath-actuated inhaler to deliver a proper dose of medication may be compromised if the user has limited lung capacity and/or lung function.

Jet nebulizers may enable users to receive medication using their normal breathing patterns and, thus, may deliver medication without requiring any forceful inhalations. However, jet nebulizers are often inefficient. The devices may nebulize more medication than can be inhaled during a single inspiratory effort. As such, significant portions of the nebulized medication may be lost during the expiratory cycle of a user's breathing pattern.
EP 2 432 536 B1 discloses a rotary cassette system for a dry powder inhaler.
US 2007/240712 A1 discloses a variable dose inhalation device.
US 2014/261414 A1 discloses an inhalation device, control method, and computer program.
EP 1 083 952 B1 discloses a device for delivery of pharmaceuticals and drugs.
EP 2 521 584 A1 discloses a method and device for delivering a pharmaceutical to the airway of a human or animal patient.

### SUMMARY

The invention is defined by claim 1 of the appended set of claims.

An inhalation device for delivering medication to a user may include a mouthpiece, a dosing chamber, and a flow channel connecting the mouthpiece to the dosing chamber. The dosing chamber may be configured to deliver the medication to the user via the mouthpiece. The inhalation device may include an electronically driven vibratory element, a sensor system configured to generate a pressure signal indicative of air flow through the flow channel, and a controller. The controller may be configured to receive the pressure signal from the sensor system. The sensor system may include an atmospheric pressure sensor (*e.g.,* a micro-electrical mechanical (MEMS) pressure sensor) and/or a differential pressure sensor. The pressure signal may include an absolute air pressure measurement. The controller may be further configured to generate a flow signal using the pressure signal, where the flow signal is an averaged output of the pressure signal. The controller may be configured to continuously determine atmospheric pressure using the pressure signal during times of no user activity (*e.g.,* between breaths), determine an average atmospheric pressure over time, and store the average atmospheric pressure in memory.

The controller may be configured to perform an inhalation detection procedure to determine a plurality of successful inhalations. The controller may be configured to activate and deactivate the vibratory element. For example, the controller may be configured to generate a trigger signal to control timing of operation of the electronically driven vibratory element to release medication into the dosing chamber and out to the patient based on the plurality of successful inhalations.

For example, the controller configured to determine whether a slope of a first cycle of the flow signal is above a predetermined slope threshold, and enter an armed state when the slope of the first cycle of the flow signal exceeds the predetermined slope threshold. The controller may be configured to start a timer when entering the armed state. In the armed state, the controller may be configured to calculate inhalation volume (volume of air through the flow channel) using the first cycle of the flow signal, determine that the inhalation volume of the first cycle exceeds an inhalation volume threshold, determine that a pressure measurement of the first cycle of the flow signal returns within a threshold of the atmospheric pressure, determine that a slope of a second cycle of the flow signal is above the predetermined slope threshold and determine that a pressure measurement of the second cycle of the flow signal exceeds a pressure threshold, and generate a trigger signal to cause the electronically driven vibratory element to release medication into the dosing chamber and out to the patient based on the slope of the second cycle of the flow signal being above the predetermined slope threshold and based on the pressure measurement of the second cycle of the flow signal exceeding the pressure threshold. The controller may be configured to exit the armed state when the inhalation volume does not exceed the inhalation volume threshold or exit the armed state when the inhalation slope does not exceed the predetermined slope threshold.

The controller may be configured to confirm a user inhalation using a first cycle of the flow signal, advance a blister pack comprising doses of medication using a second cycle of the flow signal, and generate a trigger signal to cause the electronically driven vibratory element to release a dose of medication from the blister pack into the dosing chamber using a third cycle of the flow signal. The controller may be configured to confirm a user inhalation and advance a blister pack comprising doses of medication using a first cycle of the flow signal, and generate a trigger signal to cause the electronically driven vibratory element to release a dose of medication from the blister pack into the dosing chamber using a second cycle of the flow signal.

### BRIEF DESCRIPTION OF THE DRAWINGS

FIG. 1A is a front perspective view of an example inhalation device.
FIG. 1B is a side perspective view of the example inhalation device of FIG. 1A.
FIG. 2 is a block diagram of an example inhalation device.
FIG. 3 is an interior perspective view of an example inhalation device.
FIG. 4 is a diagram of an example pressure signal received by a control circuit from an inhalation device.
FIG. 5 is a diagram of an example system including an inhalation device.
FIG. 6 is a flow diagram of an example inhalation detection procedure performed by an inhalation device.

### DETAILED DESCRIPTION

The present disclosure describes an apparatus, system, and method relating to an inhalation device that may activate the release of medication based on a user's breathing pattern, which may be a natural inhalation and exhalation pattern. The medication may be in the form of a dry powder, which may be stored in a blister strip. Each dose of dry powder medication may be stored in a particular blister within the blister strip. The inhalation device may be referred to as a tidal inhaler and may be configured to time the release of the medication during the user's inhalation such that at least a significant portion of the released medication is capable of being inhaled during the inhalation. As such, the inhalation device may be configured to efficiently deliver medication to the user while reducing or eliminating user errors sometimes associated with other devices, such as MDIs.

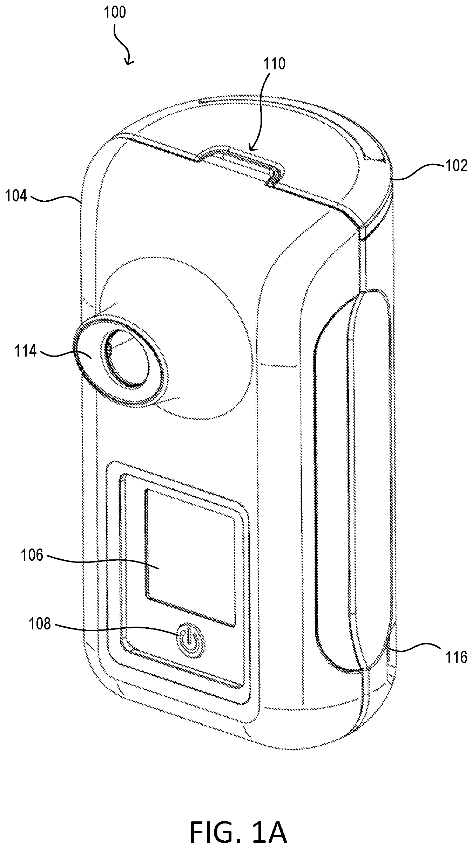

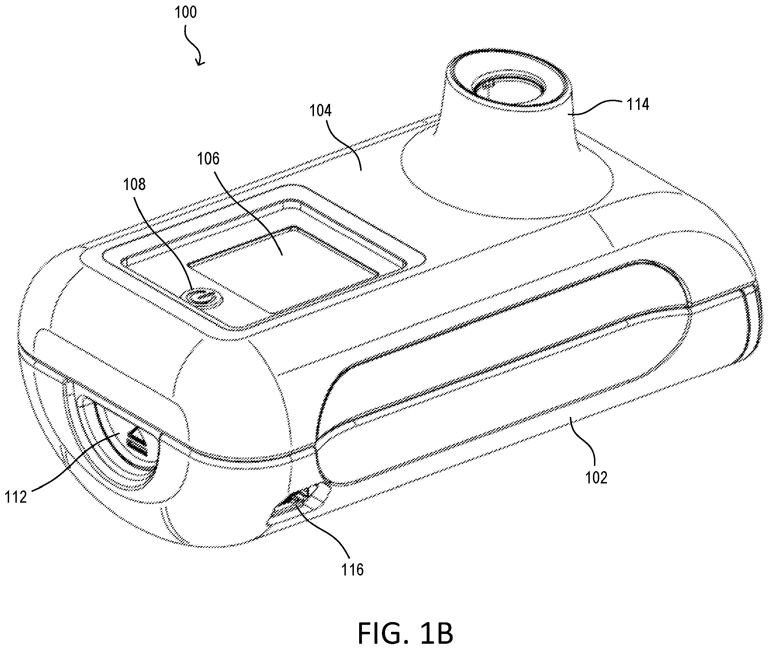

FIG. 1A is a front perspective view of an example inhalation device 100. FIG. 1B is a side perspective view of the example inhalation device 100 of FIG. 1A. The inhalation device 100 may be an active dry powder inhaler. The inhalation device 100 may provide breath activated dosing of medication by means of a plurality of tidal breaths (*e.g.,* consecutive inhalations and exhalations into and out of the inhalation device 100) and/or "pipe smoking" breaths (*e.g.,* consecutive inhalations through the inhalation device 100, and exhalations with the user's mouth removed from the device 100). The inhalation device 100 may include a main body 102 and medication cartridge 104. The main body 102 may interlock with the medication cartridge 104, for example, such that the medication cartridge 104 is a removable and replaceable from the main body 102. The medication cartridge 104 may include a plurality of doses of medication (*e.g.,* 30 doses). The medication cartridge 104 may be removed and replaced when it runs out of medication, and a new medication cartridge may be installed on the main body 102 of the inhalation device 100. As such, the main body 102 may be used with a plurality of medication cartridges 104.

The main body 102 may include one or more of the internal components of the inhalation device 100. For example, the main body 102 may include a control circuit, memory, a communication circuit, a user interface, and/or a power supply for the inhalation device 100. The main body 102 may include a power button 108 for the user to turn on/off the inhalation device 100. The main body 102 may include a power and/or data port 116 that may be used to charge the power supply of the inhalation device 100 and/or transfer data to and/or from the inhalation device 100. The main body 102 may also include an additional power and/or data port (not shown) on the side of the main body 102 opposite the power and/or data port 116 (*e.g.,* the port 116 may be used for data, while the other port opposite the port 116 may be used for power, or vice versa). The main body 102 may include a graphical user interface 106 that projects through the medication cartridge 104 when the main body 102 and medication cartridge 104 are interlocked. The main body 102 may include a release mechanism 112 that is used to release a medication cartridge 104 from the main body 102, for example, when the medication cartridge 104 runs out of medication.

The medication cartridge 104 may include a mouthpiece 114. The medication cartridge 104 may also include an air intake/exhaust port 110, which may allow for airflow between the mouthpiece 114, through the medication cartridge 104 (*e.g.,* and in turn the medication stored within), and the intake/exhaust port 110. As such, air may flow into the port 110 when a user inhales through the mouthpiece 114, and air may flow out of the port 110 when a user exhales through the mouthpieces 114. Accordingly, the inhalation device 100 may be operated as a tidal inhaler, where a user inhales through and exhales into the inhalation device 100 to receive medication. Alternatively, the inhalation device 100 may be operated as a "pipe" inhaler, where a user inhales through the inhalation device 100 but exhales with the mouthpiece away from their mouth. For example, the inhalation device 100 (*e.g.,* the medication cartridge 104) may include a one-way valve that allows for a user to inhale through the intake/exhaust port 110 and the mouthpiece 114, but prevents the user from exhaling into the mouthpiece 114 and through the intake/exhaust port 110. In one or more examples, the inhalation device 100 may be configured to operate as a tidal inhaler or as a pipe inhaler, for example, by configuring the activation of the one-way valve. Finally, the main body 102 may include a recess to accommodate the intake/exhaust port 110, for example, as shown in FIG. 1A-B.

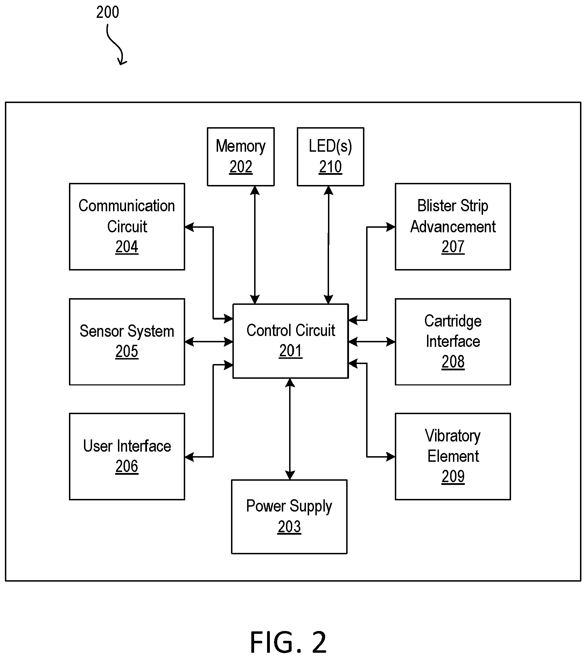

FIG. 2 is a block diagram of an example inhalation device 200. The inhalation device 200 may be an example of the inhalation device 100 of FIGS. 1A-B. The inhalation device 200 may include a control circuit 201, memory 202, a power supply 203, a communication circuit 204, a sensor system 205, a user interface 206, a blister strip advancement mechanism 207, a medication cartridge interface 208, and a vibratory element 209. The control circuit 201 may include one or more of a processor (*e.g.,* a microprocessor), a microcontroller, a programmable logic device (PLD), a field programmable gate array (FPGA), an application specific integrated circuit (ASIC), or any suitable controller or processing device.

The control circuit 201 may be configured to control the operation of one or more of the components of the inhalation device 200. For example, the control circuit 201 may be configured to receive a pressure signal from the sensor system 205. The control circuit 201 may be configured to activate and deactivate the vibratory element 209. The control circuit may be configured to perform an inhalation detection procedure to determine a plurality of successful inhalations (e.g., the inhalation detection procedure 500). The control circuit 201 may be configured to generate a trigger signal to control timing of operation of the electronically driven vibratory element 209 to release medication into a dosing chamber of the inhalation device 200 and out to the patient based on the plurality of successful inhalations.

The memory 202 may be communicatively coupled to the control circuit 201 for the storage and/or retrieval of, for example, operational settings, sensor data, *etc.* of the inhalation device 200. The memory 202 may be implemented as an external integrated circuit (IC) or as an internal circuit of the control circuit 201. The power supply 203 may generate a direct-current (DC) supply voltage Vcc for powering the control circuit 201 and the other low-voltage circuitry or high-voltage circuitry (*e.g.,* the vibratory element 209) of the inhalation device 200. The communication circuit 204 may include a wired and/or wireless communication circuit. The communication circuit 204 may be used for transmitting and/or receiving radio-frequency (RF) signals, for example, via an antenna (not shown). For example, the wireless communication circuit 204 may include an RF receiver, an RF transmitter, and/or an RF transceiver. The communication circuit 204 may transmit via a proprietary communication protocol, such as Wi-Fi, Bluetooth® *(e.*g*.,* Bluetooth Low Energy), ZIGBEE, Thread, and/or a different proprietary protocol.

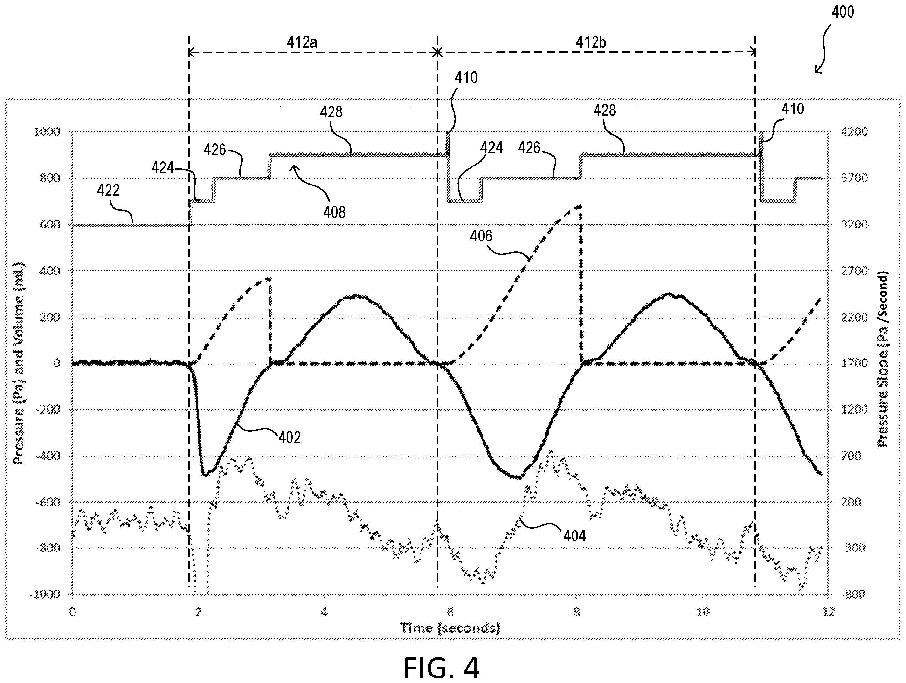

The sensor system 205 may include one or more atmospheric pressure sensors, such as micro-electrical mechanical (MEMS) pressure sensors. The MEMS pressure sensor may provide for a small, low cost sensor, although some MEMS pressure sensors may be "noisy". As such, the inhalation device 200 may perform a plurality of calculations using a pressure signal received from the sensor system 205 to accurately determine whether or not a user is breathing through the inhalation device 200, for example, as described with reference to FIG. 4-6. Alternatively or additionally, the sensor system 205 may include one or more differential pressure sensors.

At least a portion of the sensor system 205 may be in fluid communication with a flow channel between the mouthpiece and the air intake/exhaust port of the inhalation device 200. For example, the sensor system 205 may be located in the main body of the inhalation device, and may be in fluid communication with the flow channel between the mouthpiece and air intake/exhaust port of the medication cartridge. For example, the main body may include a port (*e.g.,* port 318) and the medication cartridge may include a port (*e.g.,* port 316) that resides between the mouthpiece and air intake/exhaust port of the medication cartridge. The combination of the port of the main body and the port of the medication cartridge may provide fluid communication between the sensor system 205 and the flow channel. Alternatively or additionally, the sensor system 205 may be in fluid communication through the use of a tube that is connected to the sensor system 205 and the flow channel of the medication cartridge.

The sensor system 205 may be configured to generate a pressure signal indicative of air flow through the flow channel of the inhalation device 200. The sensor system 205 may provide the pressure signal to the control circuit 201. The pressure signal may include an absolute air pressure measurement and/or unfiltered pressure measurement (*e.g.,* gauge pressure). In some embodiments, the sensor system 205 may include one or more pressure sensors that are used to measure pressure within the flow channel of the inhalation device, and one or more pressure sensors that are dedicated to measuring atmospheric pressure.

The user interface 206 may include a display (*e.g.,* the graphical user interface 106) and one or more actuators (*e.g.,* the power button 108). The display may comprise, for example, a liquid crystal display (LCD) screen. The display may be backlit by one or more lights sources (*e.g.,* white backlight LEDs). The display may be configured to display patient feedback information and/or operational characteristics of inhalation device 200 such as, for example, dose information (*e.g.,* dose reminders, dose completion/incomplete indications, dose progress indicators, dose counters, *etc.*)*,* medication cartridge information (*e.g.,* drug, doses remaining, expiry date, medication cartridge attachment messages, *etc.*)*,* battery charge level, communication and data transfer information, *etc.*

The inhalation device 200 may include one or more indicators, such as light-emitting diodes (LEDs) 210 or an organic LED (OLED) screen. The LEDs 210 may include a plurality of LEDs that are different colors. The control circuit 201 may be configured to operate the LEDs 210 to signal information to the user, for example, by illuminating an LED, flashing an LED, and/or the like. For example, the control circuit 201 may illuminate an LED of a particular color (*e.g.,* blue) to indicate that inhalation has been detected. The control circuit 201 may illuminate an LED of a different color (*e.g.,* green) to indicate that a dose of medication is complete. The control circuit may illuminate an LED of another color (*e.g.,* amber) to indicate that there is an error. The control circuit may operate an LED to indicate states of the inhalation device 200 and/or indicate a particular operation of the device 200 (*e.g.,* illuminate or flash an LED when the vibratory element 209 is activated). In some examples, the control circuit 200 may illuminate (*e.g.,* or flash) an LED as a cue to the user to inhale, and stop illuminating the LED when the dose is complete and the user may stop inhaling.

The inhalation device 200 may include medication that is packaged in a blister strip. Accordingly, the blister strip advancement mechanism 207 of the inhalation device 200 may be configured to advance the blister strip to prepare a dose of medication for delivery to the patient. For example, the blister strip may include a plurality of blisters (*e.g.,* dimples) sealed with a cover (*e.g.,* a piece of aluminum, paper, and/or plastic), where one or a plurality of blisters equate to a dose of medication for a user. The blister strip advancement mechanism 207 may be configured to remove the cover to expose the medication in a blister and move the blister into position for delivery to the user. As described in more detail herein, the blister strip advancement mechanism 207 may move a blister into position adjacent the dosing chamber and/or the vibratory element 209. The vibratory element 209 may be configured to vibrate and/or acoustically levitate medication out of the blister and into a dosing chamber of the inhalation device 200, such that the medication may pass through the dosing chamber nozzles and into the flow channel for inhalation by the user. The vibratory element 209 may, for example, include a piezoelectric transducer.

The medication cartridge interface 208 may be mechanical and/or electrical in nature. The medication cartridge interface 208 may be configured to create a secure connection between a main body and a medication cartridge of the inhalation device 200 (*e.g.,* the main body 102 and the medication cartridge 104 of the inhalation device 100). The medication cartridge interface 208 may include a sensor that provides a signal to the control circuit 201 to indicate that the medication cartridge is properly connected to the main body of the inhalation device 200. The medication cartridge interface 208 may also include internal memory, and for example, store cartridge information, such as dose information (*e.g.,* number of remaining doses).

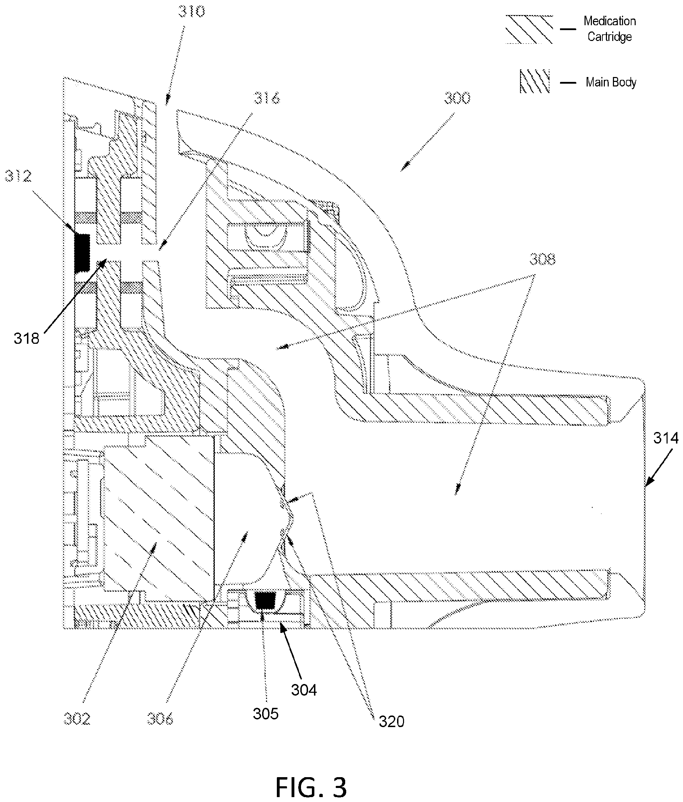

FIG. 3 is an interior perspective view of an example inhalation device 300. The inhalation device 300 may be an example of the inhalation device 100 and/or the inhalation device 200. The inhalation device may include a vibratory element 302, a blister 304, medication 305, a dosing chamber 306, a flow channel 308 between a mouthpiece 314 of the inhalation device and an air intake/exhaust port 310, a sensor system 312, and/or one or more dosing chamber nozzles 320. Air may travel from the air intake/exhaust port 310, through the flow channel 308, and out of the mouthpiece of the inhalation device, or vice versa. The vibratory element 302 may include a piezoelectric transducer.

The inhalation device 300 may include a main body and a medication cartridge. The main body may include the sensor system 312 and the vibratory element 302. The medication cartridge may include the mouthpiece 314, the blister 304, the medication 305, the dosing chamber 306 and one or more dosing chamber nozzles 320, the intake/exhaust port 310, and the flow channel 308. Different hash types used in FIG. 3 to help identify the distinction of the main body and medication cartridge of the inhalation device 300.

The sensor system 312 may be the entirety of the sensor system or a portion of the entirety of the sensor system. The sensor system 312 may be located in fluid communication with the flow channel 308, for example, such that the sensor 312 may generate a signal indicative of the air flow through the flow channel 308. For example, the medication cartridge may include a port 316 (*e.g.,* a hole) and the main body may also include a port 318 (*e.g.,* a hole). The ports 316, 318 may provide for fluid communication between the sensor system 312 and the flow channel 308. The sensor system 312 may provide a pressure signal to a control circuit (not shown) of the inhalation device 300, for example, that may be used to determine when a user is inhaling and/or exhaling into or through the inhalation device 300. For example, the intake/exhaust port 310 creates a restriction that may define a known resistance to airflow through the flow channel that is used to determine pressure changes within the flow channel. The pressure changes may be detected by the sensor system 312 and output as the pressure signal.

The control circuit of the inhalation device 300 may determine whether to deliver medication to the user, for example, as described in more detail herein. The control circuit may prepare a dose of medication residing within a blister for delivery to the patient, for example, by advancing a blister pack of medication via a blister strip advancement mechanism. When the blister 304 is in position below the dosing chamber 306 (*e.g.,* and adjacent the vibratory element 302), the control circuit may generate a trigger signal to control timing of operation of the vibratory element 302 to release the medication 305 into the dosing chamber 306. For example, the vibratory element 302 may vibrate and agitate the side walls of the dosing chamber 306, which may propel the medication 305 out of the blister 304. The vibratory element 302 may acoustically excite the dosing chamber 306, which for example, may induce synthetic jets on either side of the dosing chamber nozzles 314. The internal jets may mix or stir the medication 305 within the dosing chamber 306, and cause external jets to transport the medication 305 through the dosing chamber nozzles 320 and the flow channel 308 to the mouthpiece 314 of the inhalation device 300. Accordingly, the control circuit may time the activation of the vibratory element 302 such that the vibratory element 302 causes the release of the medication 305 into the dosing chamber 306 while a user is inhaling through the mouthpiece 314.

FIG. 4 is a diagram 400 of example signals determined by a control circuit of an inhalation device (*e.g.,* the inhalation device 100, the inhalation device 200, and/or the inhalation device 300) using a pressure signal received from a sensor system of the inhalation device. The diagram 400 may include a flow signal 402, a pressure rate-of-change (*e.g.,* slope) signal 404, a volume signal 406, and a signal 408 representing the state of the inhalation device.

The sensor system may generate a raw pressure signal (not shown) and provide the raw pressure signal to the control circuit of the inhalation device. The raw pressure signal may be in terms of absolute pressure (*e.g.,* in units of Pascals). The control circuit may receive the raw pressure signal from the sensor system. The control circuit may determine an atmospheric pressure level (not shown) using the raw pressure signal during times of inactivity (*e.g.,* when the user is not inhaling/exhaling into the device). For example, the control circuit may determine the atmospheric pressure by averaging the raw pressure signal over time, by applying a low-pass filter to the raw pressure signal, and/or the like. The control circuit may continuously determine the atmospheric pressure or determine the atmospheric pressure after determining that the slope signal 404 exceeds a predetermined slope threshold. The atmospheric pressure level may be used to determine a zero level of pressure for other calculations.

The control circuit may determine atmospheric pressure by accounting for the asymmetric inhale/exhale airway resistance and by discriminating an exhale signal that can vary from a reasonably healthy exhale (p > pₐₜₘ) and a COPD or a "pipe smoking" exhale where the exhale pressure is closer to the atmospheric pressure (*e.g.,* near the noise value of the pressure sensor since, for example, there is no exhalation into the inhalation device when "pipe smoking" occurs).

The control circuit may generate an unfiltered pressure signal (not shown) using the raw pressure signal and the atmospheric pressure, for example, so that the control circuit may account for changes in atmospheric pressure. The unfiltered pressure signal may be referred to as gauge pressure. For example, the control circuit may generate the unfiltered pressure signal by subtracting the atmospheric pressure level from the raw pressure signal, so that changes in atmospheric pressure are accounted for when making other calculations. As such, the unfiltered pressure signal may be indicative of airflow through the inhalation device (*e.g.,* through the flow channel of the inhalation device), for example, regardless of any atmospheric pressure changes. For example, the unfiltered pressure signal may provide a gauge pressure that is related to flow rate by the turbulent airflow equation, gauge pressure = (Flow Rate x Flow Resistance)².

The control circuit may generate the flow signal 402. The flow signal 402 may be a "less noisy" version of the unfiltered pressure signal. As such, the flow signal 402 may be a pressure signal. The control circuit may generate the flow signal 402 by averaging the unfiltered pressure signal over time, by applying a low-pass filter to the unfiltered pressure signal, by applying an adaptive filter algorithm to the unfiltered pressure signal, and/or the like. For example, the control circuit may sample the unfiltered pressure signal at a sampling rate (*e.g.,* 100 Hz) to generate the flow signal 402. Accordingly, the control circuit may generate the flow signal 402 using the unfiltered pressure signal by sampling the unfiltered pressure signal over time, such that the flow signal 402 is an averaged output of the unfiltered pressure signal. In some embodiments, the control circuit may determine the flow signal 402 by sampling the unfiltered pressure signal while taking into consideration the resistance of the flow channel, and add the samples up over time to determine the flow signal 402. Further, the control circuit may scale the unfiltered pressure signal with a resistance factor associated with the flow channel (*e.g.,* the intake/exhaust port) in a non-linear fashion when generating the flow signal 402.

The control circuit may determine one or more metrics using the flow signal 402 *(e.g.,* and/or the raw pressure signal or the unfiltered pressure signal). The control circuit may determine a pressure rate-of-change (*e.g.,* slope) signal 404, for example, based on flow signal 402 and time. The control circuit may determine the volume signal 406, for example, using the flow signal 402, time, flow resistance associated with the flow channel, and the atmospheric pressure. The volume signal 406 may be indicative of the volume of air that a user causes to move through the mouthpiece of the inhalation device. The control circuit may store in memory the raw pressure signal, the unfiltered pressure signal, the flow signal 402, the slope signal 404, the volume signal 406, the atmospheric pressure (*e.g.,* an average atmosphere pressure), and/or any other signal derived using the raw pressure signal, the unfiltered pressure signal, and/or the flow signal 402.

The flow signal 402 may be defined by a plurality of cycles, such as cycles 412A and 412B. A cycle of the flow signal 402 may include a negative pressure portion (*e.g.,* negative gauge pressure portion) and a positive pressure portion (*e.g.,* positive gauge pressure portion), for example, when performing tidal breathing (*e.g.,* verse just a negative portion if pipe smoking is performed). For example, a cycle of the flow signal 402 may start at a zero-crossing when the flow signal 402 becomes a negative pressure, cross zero into a positive pressure, and then return back to zero again *(e.g.,* before subsequently becoming a negative pressure again). The negative pressure portion of a cycle of the flow signal 402 and positive pressure portion of the same cycle of the flow signal 402 may be asymmetrical (*e.g.,* the pressure may different for an inhalation as compared to a corresponding exhalation of the same flow). For example, the positive pressure portion (*e.g.,* which may be caused by exhalation through the inhalation device) may be greater than the negative pressure portion of the cycle of the flow signal 402 (*e.g.,* which may be caused by inhalation through the inhalation device).

The control circuit may determine whether or not a user is inhaling and/or exhaling using one or more the of the raw pressure signal, the unfiltered pressure signal, the flow signal 402, the slope signal 404, the volume signal 406, an atmospheric pressure measurement, and/or any other signal derived using the raw pressure signal. For example, the control circuit may determine a successful inhalation of a user based on the slope signal 404, the volume signal 406, and/or the flow signal 402. A successful inhalation is typically characterized, for example, by a greater pressure rate-of-change than environmental changes (*e.g.,* due to weather, elevation change, *etc.*) and a relative total volume.

The signal 408 representing the state of the inhalation device may include tiers that represent different states of the inhalation device (*e.g.,* as described herein). For example, 422 may correlate with an unarmed and detecting state of the inhalation device, when for example, a medication cartridge is connected to the main body of the inhalation device (*e.g.,* 606 of the inhalation detection procedure 600, described herein). The inhalation device may be triggered to enter 422 after the medication cartridge is connected to the main body, before which, the inhalation device may be in an unarmed and non-detecting state (*e.g.,* 602 of the inhalation detection procedure 600). At 422, the inhalation device may determine whether the slope signal 404 exceeds a threshold or is within a predetermined slope range (*e.g.,* a rate-of-change of the flow signal 402 exceeds a threshold or is within the predetermined slope range).

If the inhalation device determines that the slope signal 404 exceeds the threshold or is within the predetermined slope range, the inhalation device may enter 424. 424 may correlate to the armed state of the inhalation device where the control circuit determines whether the volume signal 406 exceeds a threshold (*e.g.,* 610 of the inhalation detection procedure 600). If the inhalation device determines that the volume signal 406 exceeds the threshold, the inhalation device may enter 426. 426 may correlate to the armed state of the inhalation device where the control circuit determines whether the pressure of the flow signal 402 returns to a threshold of atmospheric pressure (*e.g.,* 612 of the inhalation detection procedure 600).

If the inhalation device determines that the pressure of the flow signal 402 returns to the threshold of atmospheric pressure (*e.g.,* the user stops inhaling and/or begins to exhale), the inhalation device may enter 428. At 428 the inhalation device may determine whether a slope of the flow signal (*e.g.,* a second cycle of the flow signal) is above the slope threshold or is within the predetermined slope range, and determine whether a pressure measurement of the flow signal (*e.g.,* the second cycle of the flow signal) exceeds a pressure threshold (*e.g.,* 614 of the inhalation detection procedure 600). If the inhalation device determines that the slope of the flow signal is above the slope threshold or is within the predetermined slope range, and determines that the pressure measurement of the flow signal exceeds the pressure threshold, the inhalation device may enter 410.

At 410, the inhalation device may generate a trigger signal to control timing of operation of the vibratory element to release medication into the dosing chamber (*e.g.,* the transition between 614 and 610 of the inhalation detection procedure 600) and return to 424. The inhalation device may proceed through states 424-410 until the dosing regimen is complete or a measurement (*e.g.,* based on the flow signal 402, the slope signal 404, and/or the volume signal 406) does not meet or exceed the associated threshold or range. The inhalation device may, for example, start a timer after 410. When the timer expires, the inhalation device may revert to state 422 so that the inhalation device does not determine that a non-breathing atmospheric disturbance is above the threshold (e.g., so that a non-breathing atmospheric pressure disturbance is not registered as an inhalation).

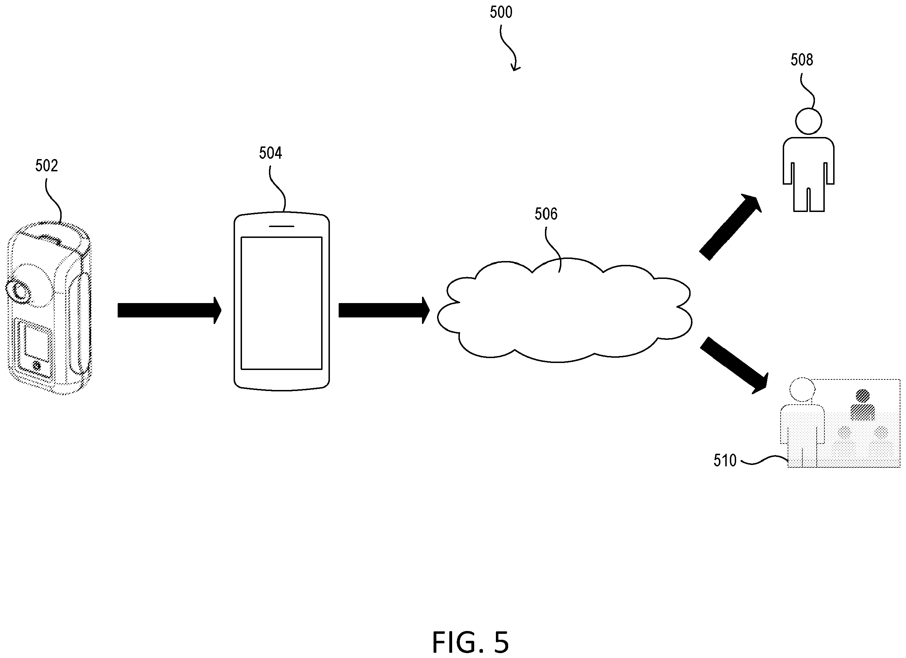

FIG. 5 is a diagram of an example system 500 including an inhalation device 502. The system 500 may include the inhalation device 502, a mobile device 504, a public and/or private network 506 (*e.g.,* the Internet, a cloud network), a health care provider 508, and a third party 510 (*e.g.,* friends, family, pharmaceutical company, *etc.*)*.* The inhalation device 502 may be an example of the inhalation device 100 and/or the inhalation device 200. The inhalation device 502 may transfer data to the mobile device 504, such as, for example, inhalation data (*e.g.,* pressure signals, flow signals, volume signals, *etc.*)*,* data generated or determined based on inhalation data, dosage information, *etc.* The inhalation device 502 may receive data from the mobile device, such as, for example, program instructions, operating system changes, dosage information, alerts or notifications, *etc.*

The mobile device 504 may include a smart phone (*e.g.,* an iPhone® smart phone, an Android® smart phone, or a Blackberry® smart phone), a personal computer, a laptop, a wireless-capable media device (*e.g.,* MP3 player, gaming device, television, a media streaming devices (*e.g.,* the Amazon Fire TV, Nexus Player, *etc.*)*, etc.*)*,* a tablet device (*e.g.,* an iPad® hand-held computing device), a Wi-Fi or wireless-communication-capable television, or any other suitable Internet-Protocol-enabled device. For example, the mobile device 504 may be configured to transmit and/or receive RF signals via a Wi-Fi communication link, a Wi-MAX communications link, a Bluetooth® or Bluetooth Smart communications link, a near field communication (NFC) link, a cellular communications link, a television white space (TVWS) communication link, or any combination thereof.

The mobile device 504 may transfer data through the public and/or private network 506 to the health care provider 508 and/or one or more third parties 510 (*e.g.,* friends, family, pharmaceutical company, *etc.*)*.*

An inhalation device (*e.g.,* the inhalation device 100, the inhalation device 200, and/or the inhalation device 502) may be configured to determine and analyze a plurality of signals received from a pressure sensor, for example, over a plurality of different cycles, to make a strong estimation of human breathing. Once the inhalation device determines that the signal(s) or measurement calculated using the signal(s) appear to be indicative of human breathing (*e.g.,* are above thresholds, within ranges, *etc.,* for example, as described herein), the inhalation device may prepare and then deliver medication to the user during their natural breathing cycle. For example, the inhalation device may receive and/or determine based on received signals one or more pressure measurements, such as, but not limited to flow (*e.g.,* gage pressure), pressure rate-of-change, volume, *etc.* Thereafter, the inhalation device may determine whether the measurement(s) has characteristics commensurate with human breathing (*e.g.,* are above thresholds, within ranges, *etc.,* for example, as described herein). For example, the inhalation device may use a plurality of the measurements in any order and combination to provide a higher confidence that the signals measured by the pressure sensor are indicative of human breathing. After determining that the measurement(s) has characteristics commensurate with human breathing, the inhalation device may confirm inhalation, prepare blister pack, and/or generate one or a plurality of trigger signals to drive the vibratory element. Accordingly, through the use of one or more measurements, the inhalation device may reduce the instances of false triggers that result in the preparation of medication when a user is not breathing through the inhalation device.

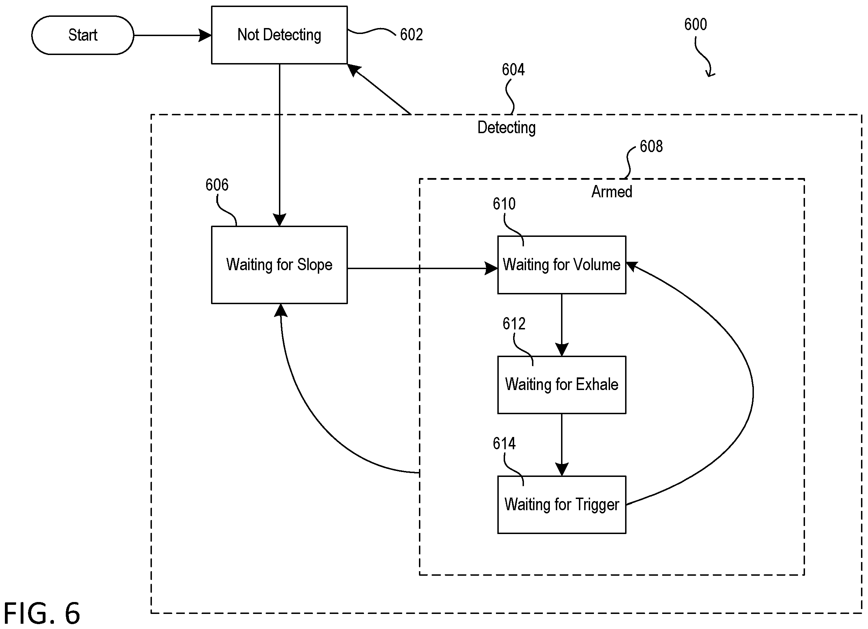

For example, FIG. 6 is a flow diagram of an example inhalation detection procedure 600 performed by an inhalation device (*e.g.,* the inhalation device 100, the inhalation device 200, and/or the inhalation device 502). The inhalation detection procedure 600 may initiate when the inhalation device is powered on. At 602, the inhalation device may be in a non-detecting state and not receive a pressure signal from the sensor system (*e.g.,* is not detecting whether or not a user is inhaling through/exhaling into the inhalation device). For example, the inhalation device may not receive a raw pressure signal if no medication cartridge is attached to the inhalation device.

The control circuit may use a plurality of cycles of a flow signal (*e.g.,* the flow signal 402) to determine successful inhalation of a user and to provide medication to the user. For example, the control circuit may be configured to confirm a user inhalation using a first cycle of the flow signal. The control circuit may advance a blister pack comprising doses of medication using a subsequent (*e.g.,* a second) cycle of the flow signal. The control circuit may generate a trigger signal to cause the vibratory element to release a dose of medication from a blister of the blister pack into the dosing chamber using one or more subsequent cycles of the flow signal.

The inhalation device may enter a detecting state 604 and receive the pressure signal *(e.g.,* a raw pressure signal) from the sensor system, which for example, may occur once a medication cartridge is attached to the inhalation device. At 606, the inhalation device may be in an unarmed state and be receiving the raw pressure signal from the sensor system. The inhalation device may determine an atmospheric pressure measurement (*e.g.,* an averaged atmospheric pressure measure) during times of no user activity (*e.g.,* at 606) using the raw pressure signal. The inhalation device may determine atmospheric pressure since user inhalations work against the atmospheric pressure, which for example, may vary according to altitude and weather conditions. The inhalation device may store the average atmospheric pressure measurement in memory. The inhalation device may measure and store the average atmospheric pressure while it is waiting for the slope of the flow signal to exceed a predetermined slope threshold, or may continuously measure and store the average atmospheric pressure measurement.

The inhalation device may determine an unfiltered pressure signal using the raw pressure signal and the average atmospheric pressure measurement, for example, as described herein. The inhalation device may determine the flow signal using the unfiltered pressure signal, for example, as described herein. Accordingly, the flow signal may be a less noisy indication of pressure through the flow channel of the inhalation device and may take into account changes of atmospheric pressure. The inhalation device may use the flow signal (*e.g.,* directly or indirectly) as a baseline from which to identify changes in pressure associated with someone breathing or variations in altitude or weather conditions. As such, the flow signal, which takes into account the atmospheric pressure, may be used as a baseline to determine successful inhalations.

The control circuit may be configured to determine whether a slope of a first cycle of the flow signal is above a predetermined slope threshold (*e.g.,* above 250 pa/sec) or is within a predetermined slope range (*e.g.,* between 250 to 3,700 pa/sec) at 606. The control circuit may determine the slope of the first cycle using the flow signal or using a slope signal *(e.g.,* the slope signal 404) that is derived using the flow signal. The control circuit may be configured to enter an armed state 608 when the slope of the first cycle of the flow signal exceeds the predetermined slope threshold or is within the predetermined slope range. For example, the control circuit may determine whether the flow signal indicates a pressure rate-of-change that is indicative of user inhalation (*e.g.,* or exhalation if positive pressure rate-of-change is detected).

In the armed state 608, the control circuit may be configured to calculate inhalation volume using the first cycle of the flow signal (*e.g.,* and/or a volume signal, such as the volume signal 406) at 610. The inhalation volume may be indicative of the volume of air through the flow channel of the inhalation device. If the control circuit determines that the inhalation volume of the first cycle exceeds an inhalation volume threshold *(e.g.,* 125 mL) at 610, then the control circuit may proceed to 612. At 612, the control circuit may be configured to determine whether a pressure measurement of the first cycle of the flow signal returns within a threshold of the atmospheric pressure (*e.g.,* within 15 pa). For example, the control circuit may determine whether a volume of air passes through the flow channel of the inhalation device that is indicative of user inhalation at 610. The control circuit may then determine whether the pressure measurement returns within the threshold of the atmospheric pressure (*e.g.,* within an amount of pressure of the average atmospheric pressure) that is indicative of the user exhaling and the flow signal potentially entering a subsequent cycle. Further, the control circuit may, for example, determine that the pressure measurement of the first cycle of the flow signal returns within the threshold of the atmospheric pressure when the pressure measurement crosses across the negative/positive boundary (e.g., zero-crossing) in the middle of the first cycle of the flow signal, which for example, may be indicative of a user exhaling into the inhalation device.

At 614, the control circuit may be configured to determine whether a slope of a second cycle of the flow signal is above the predetermined slope threshold or is within the predetermined slope range. For example, the control circuit may determine whether a slope *(e.g.,* a negative slope) of the flow signal exceeds a threshold or is within a range. At 614, the control circuit may also determine whether a pressure measurement of the second cycle of the flow signal exceeds the pressure threshold (*e.g.,* similar to as performed with respect to the first cycle). If the control circuit determines that the slope of the second cycle of the flow signal is above the predetermined slope threshold or is within the predetermined slope range, and that the pressure measurement of the second cycle of the flow signal exceeds the pressure threshold, the control circuit may generate the trigger signal to cause the vibratory element to release medication into the dosing chamber and out to the patient before returning to 610. The control circuit may repeat 610-614 and continue to generate trigger signals to cause the vibratory element to release medication into the dosing chamber and out to the patient until a particular dosing regimen is complete (*e.g.,* anywhere from one to a plurality of trigger signals may be used to provide an entire dose of medication to a user). If at any point in the inhalation detection procedure 600 the control circuit determines that a measurement does not exceed or meet its threshold or range, then control circuit may return to 606. The control circuit may also start a timer when entering the armed state 608 and return to 606 if the timer expires before the control circuit determines successful inhalation.

Although described with reference to a determination of inhalation using a specific pressure metric (*e.g.,* slope, volume, etc.) and an associated action based on that determination of inhalation (*e.g.,* confirm inhalation, prepare blister pack, generate trigger signal to drive the vibratory element), is should be noted that the inhalation device may be configured to use one or more different pressure metrics to cause one or more actions to be performed. For example, the inhalation device may determine that a first cycle of the flow signal is indicative of inhalation based on slope and volume, and confirm inhalation and prepare the blister pack accordingly. Thereafter, upon determining that a second cycle of the flow signal is also indicative of inhalation (*e.g.,* based on slope and volume), the inhalation device may generate the trigger signal to cause the electronically driven vibratory element to release a dose of medication from the blister pack into the dosing chamber. In another example, the inhalation device may determine that a first cycle of the flow signal is indicative of inhalation based on volume, and confirm inhalation accordingly. Thereafter, upon determining that a second cycle of the flow signal is also indicative of inhalation (*e.g.,* based on slope and volume and/or inhalation or exhalation duration characteristics, which for example, may be indicative of human breathing), the inhalation device may prepare the blister pack and generate the trigger signal to cause the electronically driven vibratory element to release a dose of medication from the blister pack into the dosing chamber.

The control circuit may determine that a user successfully inhaled through the inhalation device (*e.g.,* and exhaled, if tidal breathing is being performed) when the control circuit determines that the slope of a cycle of the flow signal exceeds the predetermined slope threshold and/or that the inhalation volume of the cycle exceeds the inhalation volume threshold (*e.g.,* and possible also that the pressure measurement of the cycle of the flow signal returns within the threshold of the atmospheric pressure). However, the control circuit may wait to trigger the release of medication until the control circuit determines multiple successful inhalations. For example, the control circuit may use one or more determined successful inhalations as checks to ensure that medication is not accidentally released upon a "false" inhalation determination. The control circuit may prepare a dose of medication upon determining a subsequent successful inhalation, for example, by advancing the blister pack and exposing the medication within a blister to the dosing chamber. The control circuit may then generate a trigger signal to cause the vibratory element to release medication into the dosing chamber and out to the patient upon determining one or more subsequent successful inhalations (*e.g.,* where a portion of the entire dose of medication may be delivered to the patient with each inhalation). The control circuit may utilize multiple triggers to cause the vibratory element to release medication into the dosing chamber and out to the patient, for example, to accommodate short inhalation times and prevent dosing during exhalation, to allow the vibratory element to cool between activations, *etc.* Alternatively, the control circuit may provide a single trigger signal to the vibratory element that causes the vibratory element to release all of the medication from a blister into the dosing chamber and out to the patient (*e.g.,* where the entirety of the dose may be delivered to the patient in one inhalation).

## Claims

1. An inhalation device (100, 200, 300) for delivering medication (305) to a user, said inhalation device comprising:
a mouthpiece (114, 314), a dosing chamber (306), and a flow channel (308) connecting the mouthpiece to the dosing chamber, wherein the dosing chamber is configured to deliver the medication to the user via the mouthpiece;
an electronically driven vibratory element (209, 302);
a sensor system (205, 312) configured to generate a pressure signal indicative of air flow through the flow channel; and
a controller (201) configured to receive the pressure signal from the sensor system, activate and deactivate the vibratory element, perform an inhalation detection procedure to determine a plurality of successful inhalations, and generate a trigger signal to control timing of operation of the electronically driven vibratory element to release medication into the dosing chamber based on the plurality of inhalations, wherein the inhalation device comprises a main body (102) and a replaceable medication cartridge (104), the main body including the sensor system and the vibratory element, and the medication cartridge including the mouthpiece, the medication, the dosing chamber, and the flow channel, and wherein the medication cartridge (104) comprises an intake/exhaust port (110, 310) configured to allow air to flow into the intake/exhaust port when a user inhales through the mouthpiece (114, 314), and air to flow out of the intake/exhaust port when a user exhales through the mouthpiece.

2. The inhalation device (100, 200, 300) of claim 1, wherein the controller (201) is configured to generate a flow signal based on the pressure signal and atmospheric pressure, wherein the flow signal comprises a plurality of cycles; and
wherein the controller is configured to generate the trigger signal to control the timing of operation of the electronically driven vibratory element (209, 302) to release medication into the dosing chamber (306) based on a slope of a cycle of the flow signal exceeding a slope threshold or a volume of a cycle of the flow signal exceeding a volume threshold for a plurality of inhalations.

3. The inhalation device (100, 200, 300) of claim 1, further comprising a one-way valve configurable to allow for a user to inhale through an intake port (110, 310) and the mouthpiece (114, 314), but prevent the user from exhaling into the mouthpiece and through the intake port.

4. The inhalation device (100, 200, 300) of claim 1, wherein the controller (201) is configured to generate a flow signal based on the pressure signal and atmospheric pressure, confirm a user inhalation using a first cycle of the flow signal, prepare a blister pack comprising doses of medication (305) using a second cycle of the flow signal, and generate a trigger signal to cause the electronically driven vibratory element (209, 302) to release a dose of medication from the blister pack into the dosing chamber (306) using a third cycle of the flow signal.

5. The inhalation device (100, 200, 300) of claim 1, wherein the controller (201) is further configured to generate a flow signal based on the pressure signal and atmospheric pressure, determine atmospheric pressure using the pressure signal during times of no user activity, determine an average atmospheric pressure over time, and store the average atmospheric pressure in memory.

6. The inhalation device (100, 200, 300) of claim 5, wherein the controller (201) is configured to determine whether a slope of a first cycle of the flow signal is above a predetermined slope threshold, and enter an armed state when the slope of the first cycle of the flow signal exceeds the predetermined slope threshold;
wherein, in the armed state, the controller is configured to:
calculate inhalation volume using the first cycle of the flow signal;
determine that the inhalation volume of the first cycle exceeds an inhalation volume threshold;
determine that a pressure measurement of the first cycle of the flow signal returns within a threshold of the atmospheric pressure;
determine that a slope of a second cycle of the flow signal is above the predetermined slope threshold and determine that a pressure measurement of the second cycle of the flow signal exceeds a pressure threshold; and
generate a trigger signal to cause the electronically driven vibratory element (209, 302) to release medication (305) into the dosing chamber (306) based on the slope of the second cycle of the flow signal being above the predetermined slope threshold and based on the pressure measurement of the second cycle of the flow signal exceeding the pressure threshold.

7. The inhalation device (100, 200, 300) of claim 6, wherein the controller (201) is further configured to start a timer when entering the armed state, and further configured to revert to an unarmed state if the timer elapses.

8. The inhalation device (100, 200, 300) of claim 6, wherein the controller (201) is further configured to exit the armed state when the inhalation volume does not exceed the inhalation volume threshold or exit the armed state when the inhalation slope does not exceed the predetermined slope threshold.

9. The inhalation device (100, 200, 300) of claim 1, wherein the controller (201) is further configured to calculate volume using the pressure signal, and configured to determine successful inhalation based on the volume being above a volume threshold.

10. The inhalation device (100, 200, 300) of claim 1, wherein the sensor system (205, 312) comprises an atmospheric pressure sensor.

11. The inhalation device (100, 200, 300) of claim 1, wherein the electronically driven vibratory element (209, 302) is configured to vibrate or acoustically levitate the medication (305) out of a blister (304) and into the dosing chamber (306).

12. The inhalation device (100, 200, 300) of claim 1, wherein the dosing chamber (306) comprises nozzles, and wherein the electronically driven vibratory element (209, 302) is configured to excite the dosing chamber such that the medication (305) exits the dosing chamber through the nozzles and into the flow channel (308).

13. The inhalation device (100, 200, 300) of claim 1, further comprising a user interface (106, 206).

14. The inhalation device (100, 200, 300) of claim 1, further comprising a communication circuit (204).

## Patentansprüche

1. Inhalationsvorrichtung (100, 200, 300) zum Abgeben von Medikamenten (305) an einen Benutzer, wobei die Inhalationsvorrichtung Folgendes umfasst:
ein Mundstück (114, 314), eine Dosierkammer (306) und einen Strömungskanal (308), der das Mundstück mit der Dosierkammer verbindet, wobei die Dosierkammer dazu konfiguriert ist, das Medikament über das Mundstück an den Benutzer abzugeben;
ein elektronisch angetriebenes Vibrationselement (209, 302);
ein Sensorsystem (205, 312), das dazu konfiguriert ist, ein Drucksignal zu erzeugen, das einen Luftstrom durch den Strömungskanal anzeigt; und
eine Steuerung (201), die dazu konfiguriert ist, das Drucksignal von dem Sensorsystem zu empfangen, das Vibrationselement zu aktivieren und zu deaktivieren, eine Inhalationserkennungsprozedur durchzuführen, um eine Vielzahl erfolgreicher Inhalationen zu bestimmen, und ein Triggersignal zu erzeugen, um die Betriebszeit des elektronisch angetriebenen Vibrationselements zum Freigeben von Medikamenten in die Dosierkammer basierend auf der Vielzahl von Inhalationen zu steuern, wobei die Inhalationsvorrichtung einen Hauptkörper (102) und eine austauschbare Medikamentenkartusche (104) umfasst, wobei der Hauptkörper das Sensorsystem und das Vibrationselement beinhaltet, und die Medikamentenkartusche, das Mundstück, das Medikament, die Dosierkammer und den Strömungskanal beinhaltet, und wobei die Medikamentenkartusche (104) eine Einlass-/Auslassöffnung (110, 310) umfasst, die dazu konfiguriert ist, zu ermöglichen, dass Luft in die Einlass-/Auslassöffnung strömt, wenn ein Benutzer durch das Mundstück (114, 314) einatmet und Luft aus der Einlass-/Auslassöffnung strömt, wenn ein Benutzer durch das Mundstück ausatmet.

2. Inhalationsvorrichtung (100, 200, 300) nach Anspruch 1, wobei die Steuerung (201) dazu konfiguriert ist, ein Strömungssignal basierend auf dem Drucksignal und dem Atmosphärendruck zu erzeugen, wobei das Strömungssignal eine Vielzahl von Zyklen umfasst; und
wobei die Steuerung dazu konfiguriert ist, das Triggersignal zu erzeugen, um die Betriebszeit des elektronisch angetriebenen Vibrationselements (209, 302) zum Freigeben von Medikamenten in die Dosierkammer (306) basierend darauf zu steuern, dass eine Steigung eines Zyklus des Strömungssignals, einen Steigungsschwellenwert überschreitet oder ein Volumen eines Zyklus des Strömungssignals einen Volumenschwellenwert für eine Vielzahl von Inhalationen überschreitet.

3. Inhalationsvorrichtung (100, 200, 300) nach Anspruch 1, ferner umfassend ein Einwegventil, das so konfiguriert werden kann, dass es einem Benutzer ermöglicht, durch eine Einlassöffnung (110, 310) und das Mundstück (114, 314) einzuatmen, jedoch verhindert, dass der Benutzer in das Mundstück und durch die Einlassöffnung ausatmet.

4. Inhalationsvorrichtung (100, 200, 300) nach Anspruch 1, wobei die Steuerung (201) dazu konfiguriert ist, ein Strömungssignal basierend auf dem Drucksignal und dem Atmosphärendruck zu erzeugen, eine Benutzerinhalation unter Verwendung eines ersten Zyklus des Strömungssignals zu bestätigen, eine Blisterpackung, die Medikamentendosen (305) umfasst, unter Verwendung eines zweiten Zyklus des Strömungssignals herzustellen, und ein Triggersignal zu erzeugen, um das elektronisch angetriebene Vibrationselement (209, 302) zu veranlassen, eine Medikamentendosis aus der Blisterpackung in die Dosierkammer (306) unter Verwendung eines dritten Zyklus des Strömungssignals freizugeben.

5. Inhalationsvorrichtung (100, 200, 300) nach Anspruch 1, wobei die Steuerung (201) ferner dazu konfiguriert ist, ein Strömungssignal basierend auf dem Drucksignal und dem Atmosphärendruck zu erzeugen, den Atmosphärendruck unter Verwendung des Drucksignals während Zeiten ohne Benutzeraktivität zu bestimmen, einen durchschnittlichen Atmosphärendruck über die Zeit zu bestimmen und den durchschnittlichen Atmosphärendruck in einem Speicher speichern.

6. Inhalationsvorrichtung (100, 200, 300) nach Anspruch 5, wobei die Steuerung (201) dazu konfiguriert ist, zu bestimmen, ob eine Steigung eines ersten Zyklus des Strömungssignals über einem vorbestimmten Steigungsschwellenwert liegt, und in einen betriebsbereiten Zustand einzutreten, wenn die Steigung des ersten Zyklus des Strömungssignals den vorbestimmten Steigungsschwellenwert überschreitet;
wobei die Steuerung in dem betriebsbereiten Zustand konfiguriert ist zum:
Berechnen eines Inhalationsvolumens unter Verwendung des ersten Zyklus des Strömungssignals;
Bestimmen, dass das Inhalationsvolumen des ersten Zyklus einen Inhalationsvolumenschwellenwert überschreitet;
Bestimmen, dass eine Druckmessung des ersten Zyklus des Strömungssignals innerhalb eines Schwellenwerts des Atmosphärendrucks zurückkommt;
Bestimmen, dass eine Steigung eines zweiten Zyklus des Strömungssignals über dem vorbestimmten Steigungsschwellenwert liegt, und Bestimmen, dass eine Druckmessung des zweiten Zyklus des Strömungssignals einen Druckschwellenwert überschreitet; und
Erzeugen eines Triggersignals, um das elektronisch angetriebene Vibrationselement (209, 302) zu veranlassen, Medikamente (305) in die Dosierkammer (306) basierend darauf freizugeben, dass die Steigung des zweiten Zyklus des Strömungssignals über dem vorbestimmten Steigungsschwellenwert liegt, und basierend darauf, dass die Druckmessung des zweiten Zyklus des Strömungssignals den Druckschwellenwert überschreitet.

7. Inhalationsvorrichtung (100, 200, 300) nach Anspruch 6, wobei die Steuerung (201) ferner dazu konfiguriert ist, beim Eintreten in den betriebsbereiten Zustand, einen Timer zu starten, und ferner dazu konfiguriert ist, in einen nicht betriebsbereiten Zustand zurückzukehren, wenn der Timer abläuft.

8. Inhalationsvorrichtung (100, 200, 300) nach Anspruch 6, wobei die Steuerung (201) ferner dazu konfiguriert ist, den betriebsbereiten Zustand zu verlassen, wenn das Inhalationsvolumen den Inhalationsvolumenschwellenwert nicht überschreitet, oder den betriebsbereiten Zustand zu verlassen, wenn die Inhalationssteigung den vorbestimmten Steigungsschwellenwert nicht überschreitet.

9. Inhalationsvorrichtung (100, 200, 300) nach Anspruch 1, wobei die Steuerung (201) ferner dazu konfiguriert ist, das Volumen unter Verwendung des Drucksignals zu berechnen, und dazu konfiguriert ist, eine erfolgreiche Inhalation basierend darauf zu bestimmen, dass das Volumen über einem Volumenschwellenwert liegt.

10. Inhalationsvorrichtung (100, 200, 300) nach Anspruch 1, wobei das Sensorsystem (205, 312) einen Atmosphärendrucksensor umfasst.

11. Inhalationsvorrichtung (100, 200, 300) nach Anspruch 1, wobei das elektronisch angetriebene Vibrationselement (209, 302) dazu konfiguriert ist, um das Medikament (305) aus einem Blister (304) heraus und in die Dosierkammer (306) hinein zu vibrieren oder akustisch schweben zu lassen.

12. Inhalationsvorrichtung (100, 200, 300) nach Anspruch 1, wobei die Dosierkammer (306) Düsen umfasst und wobei das elektronisch angetriebene Vibrationselement (209, 302) dazu konfiguriert ist, die Dosierkammer so anzuregen, dass das Medikament (305) die Dosierkammer durch die Düsen und in den Strömungskanal (308) verlässt.

13. Inhalationsvorrichtung (100, 200, 300) nach Anspruch 1, ferner umfassend eine Benutzerschnittstelle (106, 206).

14. Inhalationsvorrichtung (100, 200, 300) nach Anspruch 1, ferner umfassend eine Kommunikationsschaltung (204).

## Revendications

1. Dispositif d'inhalation (100, 200, 300) permettant d'administrer un médicament (305) à un utilisateur, ledit dispositif d'inhalation comprenant :
un embout buccal (114, 314), une chambre de dosage (306) et un canal d'écoulement (308) reliant l'embout buccal à la chambre de dosage, ladite chambre de dosage étant conçue pour administrer le médicament à l'utilisateur par l'intermédiaire de l'embout buccal ;
un élément vibratoire à commande électronique (209, 302) ;
un système de capteur (205, 312) conçu pour générer un signal de pression indiquant l'écoulement d'air à travers le canal d'écoulement ; et
un dispositif de commande (201) conçu pour recevoir le signal de pression en provenance du système de capteur, activer et désactiver l'élément vibratoire, exécuter une procédure de détection d'inhalation pour déterminer une pluralité d'inhalations réussies, et générer un signal de déclenchement pour commander la synchronisation du fonctionnement de l'élément vibratoire à commande électronique pour libérer un médicament dans la chambre de dosage sur la base de la pluralité d'inhalations, ledit dispositif d'inhalation comprenant un corps principal (102) et une cartouche de médicament remplaçable (104), le corps principal comprenant le système de capteur et l'élément vibratoire, et la cartouche de médicament comprenant l'embout buccal, le médicament, la chambre de dosage et le canal d'écoulement, et ladite cartouche de médicament (104) comprenant un orifice d'entrée/sortie (110, 310) conçu pour permettre à l'air de s'écouler dans l'orifice d'entrée/sortie lorsqu'un utilisateur inhale par l'intermédiaire de l'embout buccal (114, 314), et de l'air s'écoule hors de l'orifice d'entrée/sortie lorsqu'un utilisateur exhale à travers l'embout buccal.

2. Dispositif d'inhalation (100, 200, 300) selon la revendication 1, ledit dispositif de commande (201) étant conçu pour générer un signal d'écoulement basé sur le signal de pression et la pression atmosphérique, ledit signal d'écoulement comprenant une pluralité de cycles ; et
ledit dispositif de commande étant conçu pour générer le signal de déclenchement afin de commander la synchronisation du fonctionnement de l'élément vibratoire à commande électronique (209, 302) pour libérer le médicament dans la chambre de dosage (306) sur la base d'une pente d'un cycle du signal d'écoulement dépassant un seuil de pente ou d'un volume d'un cycle du signal d'écoulement dépassant un seuil de volume pour une pluralité d'inhalations.

3. Dispositif d'inhalation (100, 200, 300) selon la revendication 1, comprenant en outre une valve anti-retour pouvant être configurée pour permettre à un utilisateur d'inhaler par l'intermédiaire d'un orifice d'entrée (110, 310) et de l'embout buccal (114, 314), mais empêcher l'utilisateur d'expirer dans l'embout buccal et par l'intermédiaire de l'orifice d'entrée.

4. Dispositif d'inhalation (100, 200, 300) selon la revendication 1, ledit dispositif de commande (201) étant conçu pour générer un signal d'écoulement basé sur le signal de pression et la pression atmosphérique, confirmer l'inhalation d'un utilisateur à l'aide d'un premier cycle du signal d'écoulement, préparer une emballage coque comprenant des doses de médicament (305) à l'aide d'un deuxième cycle du signal d'écoulement, et générer un signal de déclenchement pour amener l'élément vibratoire à commande électronique (209, 302) à libérer une dose de médicament de l'emballage coque dans la chambre de dosage (306) à l'aide d'un troisième cycle du signal d'écoulement.

5. Dispositif d'inhalation (100, 200, 300) selon la revendication 1, ledit dispositif de commande (201) étant en outre conçu pour générer un signal d'écoulement basé sur le signal de pression et la pression atmosphérique, déterminer la pression atmosphérique à l'aide du signal de pression pendant les périodes d'inactivité de l'utilisateur, déterminer une pression atmosphérique moyenne au fil du temps et stocker la pression atmosphérique moyenne en mémoire.

6. Dispositif d'inhalation (100, 200, 300) selon la revendication 5, ledit dispositif de commande (201) étant conçu pour déterminer si une pente d'un premier cycle du signal d'écoulement est supérieure à un seuil de pente prédéfini, et entrer dans un état enclenché lorsque la pente du premier cycle du signal d'écoulement dépasse le seuil de pente prédéfini ;
dans l'état enclenché, ledit dispositif de commande étant conçu pour :
calculer le volume d'inhalation à l'aide du premier cycle du signal d'écoulement ;
déterminer que le volume d'inhalation du premier cycle dépasse un seuil de volume d'inhalation ;
déterminer qu'une mesure de pression du premier cycle du signal d'écoulement revient à l'intérieur d'un seuil de la pression atmosphérique ;
déterminer qu'une pente d'un deuxième cycle du signal d'écoulement est supérieure au seuil de pente prédéfini et déterminer qu'une mesure de pression du deuxième cycle du signal d'écoulement dépasse un seuil de pression ; et
générer un signal de déclenchement pour amener l'élément vibratoire à commande électronique (209, 302) à libérer le médicament (305) dans la chambre de dosage (306) sur la base de la pente du deuxième cycle du signal d'écoulement qui se trouve en-dessus du seuil de pente prédéfini et sur la base de la mesure de pression du deuxième cycle du signal d'écoulement qui dépasse le seuil de pression.

7. Dispositif d'inhalation (100, 200, 300) selon la revendication 6, ledit dispositif de commande (201) étant en outre conçu pour démarrer une temporisation lors de l'entrée en état enclenché, et en outre conçu pour revenir à un état non enclenché si la temporisation s'est écoulée.

8. Dispositif d'inhalation (100, 200, 300) selon la revendication 6, ledit dispositif de commande (201) étant en outre conçu pour quitter l'état enclenché lorsque le volume d'inhalation ne dépasse pas le seuil de volume d'inhalation ou pour quitter l'état enclenché lorsque la pente d'inhalation ne dépasse pas dépasser le seuil de pente prédéfini.

9. Dispositif d'inhalation (100, 200, 300) selon la revendication 1, ledit dispositif de commande (201) étant en outre conçu pour calculer le volume à l'aide du signal de pression, et conçu pour déterminer une inhalation réussie sur la base du volume qui est supérieur à un seuil de volume.

10. Dispositif d'inhalation (100, 200, 300) selon la revendication 1, ledit système de capteur (205, 312) comprenant un capteur de pression atmosphérique.

11. Dispositif d'inhalation (100, 200, 300) selon la revendication 1, ledit élément vibratoire à commande électronique (209, 302) étant conçu pour faire vibrer ou faire léviter acoustiquement le médicament (305) hors d'un emballage coque (304) et dans la chambre de dosage (306).

12. Dispositif d'inhalation (100, 200, 300) selon la revendication 1, ladite chambre de dosage (306) comprenant des buses, et ledit élément vibratoire à commande électronique (209, 302) étant conçu pour exciter la chambre de dosage de sorte que le médicament (305) sorte de la chambre de dosage par les buses et dans le canal d'écoulement (308).

13. Dispositif d'inhalation (100, 200, 300) selon la revendication 1, comprenant en outre une interface utilisateur (106, 206).

14. Dispositif d'inhalation (100, 200, 300) selon la revendication 1, comprenant en outre un circuit de communication (204).
